# EUROPEAN PATENT APPLICATION

(11) **EP 3 789 760 A1**
(43) Date of publication of application: **10.03.2021**
(21) Application number: 19195630.9
(22) Date of filing: 05.09.2019
(51) Int. Cl.: G01N 31/22, A61K 9/51, A61K 41/00, G01N 33/58, G01N 33/84

(54) **CARBON DIOXIDE GAS SENSOR**

(71) Applicant: PreSens Precision Sensing GmbH, 93053 Regensburg (DE)
(72) Inventor: MATARANGA-POPA, Larisa, 93053 Regensburg (DE); RIECHERS, Daniel, 93051 Regensburg (DE)
(74) Representative: Weickmann & Weickmann PartmbB

(57) **Abstract**

The present invention relates to a particle comprising at least one detecting compound, such as a pH-sensitive detecting compound, which may be used in a sensor and particularly in a CO₂ gas sensor. Moreover, the present invention relates to a composition comprising said particle as well as to methods for producing said particle, said composition, and said sensor.

## Description

The present invention relates to a particle comprising at least one detecting compound, such as a pH-sensitive detecting compound, which may be used in a sensor and particularly in a CO₂ gas sensor. Moreover, the present invention relates to a composition comprising said particle as well as to methods for producing said particle, said composition, and said sensor.

Optical sensors for the detection of gases, such as sulfur dioxide, ammonia or carbon dioxide, are known in the art. Usually, the acidic or alkaline nature of the gas to be detected is exploited. The transduction principle is based on a pH change within the sensor triggered by the gas to be detected. An increase or decrease of the pH value in the sensor may be detected optoelectronically on the basis of a change in the absorption, fluorescence, polarization, or luminescence signal of a pH-sensitive dye incorporated in the sensor.

A particular gas of interest is carbon dioxide. For detecting carbon dioxide an indirect method based on hydration of carbon dioxide to carbonic acid within an aqueous medium and subsequent pH detection, e.g. by pH sensitive dyes, may be used.

US 5,882,936 describes a CO₂ sensor comprising an emulsoid in a hydrophobic polymer, the micelles of the emulsoid comprising an aqueous buffer and a pH-sensitive dye. In order to produce the sensor element, a water-in-oil emulsion (W/O) of the buffer and the dye in a precursor of the hydrophobic polymer is prepared, which is then applied to a carrier material and cross-linked to yield emulsoids incorporated in a hydrophobic polymer matrix.

Alternatively, an aqueous mixture of a pH-sensitive dye and a buffer is impregnated on hydrophilic carrier particles, which particles are than incorporated in a hydrophobic polymer matrix to form a pH-sensitive sensor. Suitable dyes have an ionic or ionizable functional group, allowing for an immobilization on ionomer carrier particles.

US 4,557,900 describes an optical sensor, e.g. for CO₂ detection, comprising hydrophilic particles carrying an optical indicator in a buffered solution, the particles being dispersed in a hydrophobic polymer matrix.

For gas detection, the sensor element is in direct, physical contact with the analyte such as carbonic acid derived from carbon dioxide. Via a calibrated data processing device for sensor elements as e.g. described in US 5,882,936, the analyte concentration in the sensor matrix can be calculated from the optical signal of the sensor recorded by optoelectronics, wherein the light path between the sensor element and optoelectronics is achieved by optical fibers or a free optical path.

Since the above detection method also allows detection through transparent sterile barriers, such sensors have become popular in biotechnology, medical technology, aquaculture and food production. In many applications it is necessary to sterilize the optical sensor element either directly in a process vessel such as in a flow cell or bioreactor containing the sensor, or outside the process vessel with subsequent sterile insertion in the sterile process vessel. Conventional sterilization techniques are ethylene oxide (EO) sterilization, gamma and/or beta irradiation.

For the purpose of EO sterilization, the inside of the process vessel with the integrated sensor must at least be in diffusive contact with the surrounding so that ethylene oxide gas can reach the articles to be sterilized. This is achieved by, for example, packaging the process vessel with the integrated sensor in a bag comprising a polypropylene fabric with a mesh size < 0.2 µm. The sterilized process vessel including the sensor is then stored without further packaging. During storage, VOCs, e.g. from acetic acid-based cleaners, nitrogen oxides from the surrounding, H₂S, SO₂ or other acid gases can diffuse through the permeable packaging and irreversibly damage the pre-calibrated CO₂ sensor.

Gamma and beta irradiation of the sensor may cause a release of organic compounds (VOCs) and other pH-influencing gases such as NO₂, H₂S, CO₂ and SO₂ from packaging materials and/or the plastic containing product to be sterilized. Likewise, the released compounds may damage the pre-calibrated CO₂ sensor which may harm the sensor and/or reduce its storage stability.

A drawback of the above described state-of-the-art sensors is their low stability against conventional sterilization processes, in particular against volatile organic compounds (VOCs) and/or pH-influencing compounds being released from packaging material and/or plastic materials in contact with the sensor during and/or after irradiation or EO sterilization processes and during storage.

Considering the above, there is an urgent need of new sensors and sensor materials with improved stability, in particular against sterilization processes, which overcome the drawbacks of sensors known in the art.

Thus, the problem to be solved by the present invention is the provision of an improved optical sensor with high detection sensitivity and high stability against conventional sterilization methods, such as beta radiation, gamma radiation and/or EO gassing, and high storage stability.

It was surprisingly found that based on particles comprising a detecting compound, a hygroscopic compound and an inorganic buffer compound, an improved sensor element with high detection sensitivity and stability against pH-influencing gases such as VOCs, NO₂, H₂S, CO₂ and SO₂ can be provided.

In a first aspect, the present invention refers to a particle, comprising
(i) at least one inorganic compound having a buffer capacity of 0.1-50mmol/l, preferably 0.1-25 mmol/l, at 20°C in a saturated, aqueous solution,
(ii) at least one detecting compound, and
(iii) at least one hygroscopic compound.

The at least one inorganic compound (i) has a buffer capacity of 0.1-50 mmol/l, preferably 0.1-25 mmol/l, when present in a saturated, aqueous solution at 20 °C. According to the invention, the buffer capacity of a compound is defined as the molar amount of strong acid (e.g. HCI) or base (e.g. NaOH) necessary to change the pH of 1 liter of saturated solution of the compound in water at 20 °C by 1 pH unit. Weak buffer agents usually have a buffer capacity of 0.1- 50 mmol/l, preferably at most 25 mmol/l.

The solubility of the at least one inorganic compound (i) in water at 20 °C and pH 7 may be at most 1 g/l, preferably at most 0.5 g/l, more preferably 0.001-0.1 g/l, even more preferably 0.003-0.05 g/l.

The at least one inorganic compound (i) comprises pH-influencing moieties and particularly phosphate ions, hydrogen phosphate ions, dihydrogen phosphate ions, carbonate ions, hydrogen carbonate ions, hydrogen sulfate ions, borate ions or a mixture thereof.

In one embodiment, the inorganic compound (i) is selected from a carbonate salt, such as zinc carbonate or calcium carbonate, a phosphate salt or phosphate mineral, such as calcium phosphate or apatite, a hydrolysable metal oxide, such as magnesium oxide or zinc oxide, a layered mineral, such as a layered double hydroxide (LDH) and mixtures thereof.

In one preferred embodiment, the inorganic compound (i) is a carbonate salt, such as zinc carbonate or calcium carbonate.

In another preferred embodiment, the inorganic compound (i) is a phosphate salt or phosphate mineral, such as calcium phosphate or apatite.

In a still further embodiment, the inorganic compound (i) is a hydrolysable metal oxide, such as magnesium oxide or zinc oxide.

In a still further embodiment, the inorganic compound (i) is a layered mineral, such as a layered double hydroxide (LDH), preferably a modified layered mineral, such as an inorganically modified layered mineral, in particular an inorganically modified LDH. A modified layered mineral is modified by at least partially replacing the original interlayer anions or cations of the layered mineral by anions or cations other than the original interlayer anions or cations. Anions suitable for modification may be phosphate ions, hydrogen phosphate ions, dihydrogen phosphate ions, carbonate ions, hydrogen carbonate ions, hydrogen sulfate ions, borate ions or a mixture thereof. A preferred modified LDH is e.g. a zinc-aluminum LDH being modified by phosphate ions, carbonate ions, and/or hydrogen phosphate ions. Cations suitable for modification may be Zn, Al, Ca, Mg, Ba, Mn, Fe, Ni, and Cu ions.

The detecting compound (ii) is adapted to detect a species of interest, in particular a pH-influencing compound, such as a gas, which may influence the pH in an aqueous environment. Preferably, the detecting compound (ii) is adapted to detect CO₂, e.g. yielding carbonic acid when contacted with aqueous medium. In a preferred embodiment, the detecting compound (ii) is a pH-sensitive detecting compound, particularly a pH sensitive dye. pH sensitivity of the detecting compound means that the detecting compound reproducibly changes its characteristics, e.g. its absorption, fluorescence, polarization, or luminescence, dependent on the pH of the surrounding medium, such as water or gas, particularly water. The range in which the detecting compound preferably changes its respective characteristics preferably occurs at a pH-range around the pKa value of the compound to be detected, such as in a range of pKa - 3 ≤ pH ≤ pKa + 3, more preferably pKa - 2 ≤ pH ≤ pKa + 2, more preferably pKa - 1 ≤ pH ≤ pKa + 1. Preferably, the at least one detecting compound is pH sensitive in a range of at least ± 1 pH-unit to the pKa value of a compound to be detected. With regard to the detection of carbon dioxide (pKa (carbonic acid) ≈ 6.5), it is particularly preferred that the detecting compound (ii) is pH sensitive in a range of pH 3.5 - 10.0, preferably 5.5 - 9.5.

The detecting compound (ii) may be insoluble in water, e.g. having a solubility in water at 20°C of 0.001-0.1 g/l. Suitable detecting compounds are e.g. fluorescence and/or absorption dyes, more preferably pH-sensitive fluorescence and/or absorption dyes.

In one embodiment, the detecting compound comprises a fluorescence dye such as fluorescein, trisodium 8-hydroxypyrene-1,3,6-trisulfonate (HPTS), pyrenebutyric acid, 4-methylumbelliferone, acridine orange, disodium dihydroxypyrene disulfonate, SNAFL, SNARF, rhodamine B, aza-BODIPY, xylenol blue, m-cresol purple, thymolphthalein, carboxylfluorescein, 5'- and 6'-carboxyseminaphthofluorescein (c-SNAFL), 5'-(and 6')-carboxyseminaphthorhodafluorescein (c-SNARF), 7-hydroxycoumarin-4-acetic acid, tris(thenoyltrifluoroacetonato)europium (III), 1-hydroxypyrene-3,6,8-trisulfonic acid, neutral red, phenol red, rhodamine 6G, sulforhodamine 101, texas red hydrazide, bromocresol purple, eosin Y, 2',7'-dichlorofluorescein, oregon green, 3-substiuted 7-hydroxycoumarin or a salt or derivative thereof. Preferably, the at least one detecting compound is HPTS or a salt or derivative thereof, most preferably HPTS.

In another embodiment, the at least one detecting compound comprises an absorption dye such as thymol blue, cresol red, phenol red, cresol purple, bromocresol green, phenolphthalein, o-cresolphthalein, bromothymol blue, bromophenol blue, 2-(2,4-dinitrophenylazo)-1-naphthol-3,6-disulfonic acid or a salt or derivative thereof, preferably cresol purple, thymol blue, or a salt or derivative thereof, most preferred thymol blue, or a salt or derivative thereof, most preferred thymol blue.

The hygroscopic compound (iii) comprised in the particle according to the invention may be an organic compound, particularly an organic compound which is hydrolytically stable. A suitable hygroscopic compound is e.g. a homopolymer or copolymer, particularly a hydrophilic homopolymer or copolymer, more preferably a homopolymer or copolymer comprising ionizable and/or ionic functional groups such as carboxyl groups, quarternary amine groups, sulfo groups, or phosphonium groups.

In particular, the hygroscopic compound (iii) is selected from polyvinylpyrrolidone (PVP), polyacrylamide, (hydroxyethyl)methacrylate (HEMA), cellulose derivatives such as carboxymethyl cellulose (CMC), methyl cellulose, ethyl cellulose, and hypromellose, agarose, dextran, poly(ethylene glycol), poly(propylene glycol), polyurethane, polyacrylic acid, or copolymers deriving from at least one of a (meth)acrylic acid, (hydroxyethyl)methacrylate (HEMA), vinylpyrrolidon monomeric unit, or a mixture thereof.

The particle according to the invention may be in the form of a core-shell-particle. In a preferred embodiment, the shell comprises the at least one detecting compound (ii) and the at least one hygroscopic compound (iii), while the inorganic compound (i) preferably represents the core.

At least one detecting compound (ii) may be coupled to at least one hygroscopic compound (iii) via covalent interactions and/or coulomb interactions.

The particle may have an average particle size of up to 500 µm, preferably up to 100 µm, more preferably 0.01-10 µm, even more preferably 0.01-1 µm, as determined via conventional methods known in the art such as dynamic light scattering (DIN ISO 22412).

The amount of the at least one inorganic compound (i) present in the particle is e.g. 50-90 wt.-%, preferably 60-84 wt.-% with reference to the total weight of the particle. The amount of the at least one detecting compound (ii) present in the particle is e.g. 0.1-5 wt.-%, preferably 0.1-2 wt.-% with reference to the total weight of the particle. The amount of the at least one hygroscopic compound (iii) present in the particle is e.g. 10-30 wt.-%, preferably 15-30 wt.-% with reference to the total weight of the particle. In a preferred embodiment, the particle comprises 60-84 wt.-% of the at least one inorganic compound, 0.1-2 wt.-% of the at least one detecting compound and 15-30 wt.-% of the at least one hygroscopic compound. Additionally, the particle may further comprise adsorbed additives, such as enzymes like carbonic anhydrase to speed up the response of e.g. a CO₂-sensor.

In another embodiment, the particle may further comprise at least one reference detecting compound as described below. The at least one reference detecting compound may be present in the shell of the preferred core-shell particle together with the at least one detecting compound (ii) and the at least one hygroscopic compound (iii), while the inorganic compound (i) preferably represents the core.

In a further aspect, the present invention refers to a method for producing a particle according to the invention, comprising:
a) providing a mixture comprising at least one detecting compound (ii), at least one hygroscopic compound (iii) and at least one liquid medium,
b) applying the mixture of step a) to at least one inorganic compound (i), and
c) optionally at least partially removing the at least one liquid medium from the mixture obtained in step b),
wherein the at least one inorganic compound (i) has a buffer capacity of 0.1-50 mmol/l at 20 °C in a saturated, aqueous solution.

The liquid medium may be an organic solvent, and is preferably selected from an alcohol, such as methanol, ethanol or propanol, tetrahydrofuran, 1,4-dioxane, dichloromethane, acetonitrile, acetone, dimethylformamide, N-methylpyrrolidone, or a mixture thereof, more preferably methanol or ethanol. Preferably the liquid medium is essentially free from water, and is more preferably methanol essentially free from water.

The mixture provided in step a) may be essentially free from water. Particularly, the at least one hygroscopic compound (iii) and/or the at least one liquid medium are essentially free from water. "Essentially free from water" means that the mixture or compound has a water content of at most 0.5 wt.-%, preferably 0.001-0.1 wt.%, more preferably at most 0.05 wt.-%.

The at least one detecting compound (ii) is preferably as described above.

The at least one hygroscopic compound (iii) is preferably as described above.

In a preferred embodiment, the at least one detecting compound (ii) and/or the at least one hygroscopic compound (iii) are soluble in the liquid medium. Thus, the mixture provided in step a) preferably is in the form of a solution or colloid. *"Soluble"* in the sense of the present invention means that the compound is soluble (i.e. gives a transparent solution) in a medium at 20 °C and pH 7 to an extent of at least 0.5 g/l, preferably at least 50 g/l.

The ratio of the at least one detecting compound (ii) to the at least one hygroscopic compound (iii) in step a) may be 1:10 to 1:1, preferably 1:5 to 3:10.

In one embodiment, the mixture in step a) may further comprise at least one reference detecting compound as described below. The at least one reference detecting compound may be present in the particle of the invention in an amount of 1 to 15 wt.-% .with reference to the total weight of the particle.

The at least one inorganic compound (i) provided in step b) is as described above.

Preferably, the at least one inorganic compound (i) provided in step b) is present in the form of solid particles.

The ratio of the at least one inorganic compound (i) to the at least one hygroscopic compound (iii) in step b) is e.g. 3:10 to 1:1, preferably 1:3 to 2:5.

The ratio of the at least one inorganic compound (i) to the at least one detecting compound (ii) in step b) is e.g. 1:1 to 10:1, preferably 3:2 to 6:1.

Step b) may be performed by mixing the at least one inorganic compound (i) and the mixture provided in step a) in a reaction vessel, e.g. by stirring.

In another embodiment, step b) is performed by atomizing the mixture provided in step a) onto or around the at least one inorganic compound (i). Step b) is preferably performed by conventional means, such as spray-drying, granulation, fluid bed granulation.

Step b) may be performed at temperatures of 20-80°C.

In optional step c) the at least one liquid medium is at least partially removed from the mixture obtained in step b). Preferably, at last 90%, particularly at least 95%, more preferably at least 99% of the at least one liquid medium is removed. Removing the at least one liquid medium in step c) may be performed at elevated temperatures, such as above 20 °C, preferably at 40-60 °C. Optionally, step c) is performed at reduced pressure, e.g. below 1 bar, such as below 500 mbar, preferably at 1-140 mbar.

In one embodiment, steps b) and c) are performed sequentially. In another embodiment, steps b) and c) are combined and performed simultaneously. A suitable way to combine steps b) and c) is atomizing the mixture provided in step a) onto the at least one inorganic compound, e.g. using a conventional spray granulation method. Spray granulation may be performed at temperatures of 20-80°C and optionally under reduced pressure, thus, the liquid medium is at least partially removed during the spray process.

In the absence of step c), the mixture obtained in step b) may subsequently be ground, e.g. using a ball mill or a cryogenic mill. In the presence of step c), the mixture obtained in step c) may subsequently be ground, e.g. using a ball mill or a cryogenic mill. The average particle size may be adjusted to at most 500 µm, preferably at most 100 µm, more preferably 0.01-10 µm, even more preferably 0.01-1 µm.

In a still further aspect, the present invention refers to a composition comprising particles as described herein and (x) at least one hydrophobic polymer or (xi) at least one precursor thereof and optionally a reference detecting compound.

In one embodiment, the composition comprises particles as described herein and (x) at least one hydrophobic polymer. Preferably, the particles are incorporated in the at least one hydrophobic polymer (x).

A hydrophobic polymer (x) according to the invention particularly has a limited swelling capacity such as a swelling capacity of 0.01-10 wt.-% in water at 25 °C. The free volume of the at least one hydrophobic polymer (x) may be 0.01-0.50%, preferably 0.10-0.50% at 20°C measured according to positron annihilation lifetime spectroscopy (PALS) as known in the art. By providing a hydrophobic polymer having a swelling capacity of not more than 10 wt.-% and/or a free volume of at least 0.01%, a hydrophobic polymer having a suitable water permeability is provided.

The hydrophobic polymer (x) is preferably a cross-linked polymer. A suitable hydrophobic polymer (x) is e.g. selected from a silicone polymer such as polydimethylsiloxane (PDMS) or a fluorinated silicone polymer, and a silicone copolymer such as a PDMS copolymer, a silicone-polycarbonate copolymer or a fluorinated silicone copolymer.

Preferably, the hydrophobic polymer (x) is permeable to radiation, e.g. having a wavelength between 300 and 1300 nm. This allows for an excitation of the at least one detecting compound (ii) and/or the reference detecting compound by radiation having e.g. a wavelength between 300 and 1300 nm. Moreover, emission and/or absorption of the at least one (reference) detecting compound may be detected through the hydrophobic polymer (x).

In another embodiment, the composition comprises particles of the invention and at least one precursor (xi) capable of forming a hydrophobic polymer (x). The at least one precursor (xi) may comprise at least one monomer capable of forming a hydrophobic polymer (x) and optionally at least one initiator, at least one catalyst, at least one cross-linker or a mixture thereof.

A suitable monomer may be selected from vinyl terminated poly(dimethylsiloxane), vinylphenylmethyl terminated (phenylmethylsiloxane)-vinylphenylsiloxane copolymer, vinyl terminated diphenylsiloxane-dimethylsiloxane copolymers, vinyl terminated trifluoropropylmethylsiloxane-dimethylsiloxane copolymer, vinyl terminated nonafluorohexylmethylsiloxane-dimethylsiloxane copolymer, vinyl terminated diethylsiloxane-dimethylsiloxane copolymers, vinyl terminated ethylene-siloxane copolymer fluids, and mixtures thereof.

A suitable initiator may be selected from benzoyl peroxide, cumene hydroperoxide, and mixtures thereof.

A suitable catalyst may be selected from photo-active precious metal catalysts such as (trimethyl)methylcyclopentadienylplatinum(IV) or tris(dibutylsulfide)rhodium trichloride, or non-photoinitiated catalysts such as Karstedt's catalyst or platinum-divinyltetramethyldisiloxane complex.

A suitable cross-linker may be selected from (methylhydrosiloxane)-dimethylsiloxane copolymers, trimethylsiloxy terminated polydimethylsiloxanes, hydride terminated polydimethylsiloxanes, and mixtures thereof.

The amount of particles present in the composition of the invention may be 5-50 wt.-%, preferably 10-20 wt.-% with reference to the total weight of the composition.

The amount of at least one hydrophobic polymer (x) or precursor (xi) present in the composition of the invention may be 48-95 wt.-%, preferably 80-90 wt.-% with reference to the total weight of the composition. In one embodiment, the composition comprises 5-50 wt.-% of at least one particle of the invention and 48-95 wt.-% of at least one hydrophobic polymer (x). In another embodiment, the composition comprises e.g. 5-50 wt.-% of at least one particle of the invention and 48-95 wt.-% of at least one precursor (xi).

Additionally, the composition may comprise at least one reference detecting compound. A reference detecting compound is a detecting compound which has a characteristic property that is independent of the presence or absence of the analyte to be detected. In particular, a reference detecting compound according to the invention is a pH insensitive detecting compound, preferably a pH insensitive dye, such as a pH insensitive emission, fluorescence or absorption dye. A suitable reference detecting compound is e.g. a long luminescence lifetime dye, such as ruthenium diphenylphenanthroline, or a long luminescence lifetime pigment, such as Egyptian blue (cuprorivaite). A long luminescence lifetime is defined as having a lifetime which is significantly higher than the (fluorescence or absorption) lifetime of the at least one detecting compound (ii), such as > 5 µs at 20 °C.

In one embodiment, the at least one reference detecting compound is identical to the at least one detecting compound, wherein the detecting/reference detecting compound has an analyte-dependent and an analyte-independent property. In particular, the detecting/reference detecting compound may be excited by two differing wavelengths λ1 and λ2, wherein the emission/absorption resulting from excitation λ1 is analyte-dependent, such as pH-dependent, and the emission/absorption resulting from excitation λ2 is analyte-independent. A suitable detecting/reference detecting compound is e.g. HPTS. The respective excitation wavelengths λ1 and λ2 and the corresponding emission/absorption wavelengths depend on the detecting/reference detecting compound used.

In another embodiment, the at least one reference detecting compound differs from the at least one detecting compound. The reference detecting compound and the at least one detecting compound may be present in the same particle. Alternatively, the at least one reference detecting compound may be comprised in a particle, e.g. different from that of the invention.

Thus, the composition according to the invention may further comprise particles having at least one reference detecting particle in which the at least one reference detecting compound is preferably incorporated within at least one oxygen-impermeable, optionally cross-linked polymer such as polyacrylonitrile.

A particularly preferred reference detecting compound is a reference dye which may be excited at the same wavelength λ1 as the at least one detecting compound. However, the emitted wavelength of the reference detecting compound and the detecting compound differ from each other. Suitable reference particles are e.g. as described in EP 1 196 780 B1 which is herein incorporated by reference.

The amount of the at least one reference detecting compound present in the composition of the invention may be 0.5-20 wt.-%, preferably 2-20 wt.-% preferably 3-15 wt.-% with reference to the total weight of the composition. The weight ratio of particles comprising at least one detecting compound to particles comprising at least one reference detecting compound may be 10:1 - 1:1, preferably 2:1 - 1:1.

In one embodiment, the composition according to the invention comprises 5-50 wt.-% of particles of the invention, 2-20 wt.-% of particles comprising at least one reference detecting compound, and 48-95 wt.-% of polymer (x) or polymer precursor (xi).

In a still further aspect, the present invention refers to the use of a composition as described herein as a sensor. Preferably, the composition as described herein may be used as a gas sensor, particularly a CO₂ gas sensor.

In a still further aspect, the present invention refers to a sensor, preferably a gas sensor, particularly a CO₂ gas sensor. The sensor comprises the composition comprising at least one hydrophobic polymer (x) as described herein. In the sensor, the composition is in physical contact with the analyte such as carbonic acid derived from carbon dioxide.

The water permeability of the at least one hydrophobic polymer (x) is particularly relevant for sensor applications. At the first contact with an aqueous analyte (e.g. a cell culture medium comprising carbon dioxide), the hydrophobic polymer allows for a limited diffusion of water into the composition. Diffusing water comes into contact with the particles and partially dissolves said particles. In particular the at least one inorganic compound, thereby forms a buffered aqueous medium within the hydrophobic polymer which surrounds the partially dissolved particles. In case the compound to be detected is carbon dioxide, carbon dioxide present in the analyte can diffuse into the composition and can form carbonic acid with the water diffused therein. The resulting pH change of the buffered aqueous medium surrounding the particles can then be detected via the at least one detecting compound comprised in the particle.

Conventional sensor compositions known in the art are based on highly soluble buffer compounds, such as sodium bicarbonate or sodium hydrogen phosphate buffer systems. Moreover, prior art sensor compositions usually comprise water, e.g. in the form of emulsoids, which dissolves the buffer already during sterilization and subsequent storage. As a consequence, these sensors get easily poisoned by pH-influencing compounds present during sterilization and subsequent storage. That is, the detection ability of the detecting compound is (partially) exhausted or destroyed prior to the actual detecting application. To counteract this issue, prior art sensors frequently comprise high amounts of buffer substance in order to achieve resistance to pH-influencing compounds present prior to the actual detecting application. However, an increased buffer concentration directly reduces the sensitivity of the sensor, since the amount of dissolved buffer substance and thus the buffer capacity defines the sensitivity of a pH-sensitive sensor.

In contrast, the composition according to the invention may be (essentially) free from water prior to the actual detecting application. By this means, the at least one inorganic compound is present in solid form and does not form a buffer solution which may react with acidic compounds such as gases present during sterilization and storage of the composition. Moreover, it was surprisingly found that the particles according to the invention comprising at least one inorganic compound having a buffer capacity of 0.1-50 mmol/l at 20 °C in a saturated, aqueous solution provide a sufficiently large but mainly stationary reservoir of buffer so that the sensor composition can regenerate almost completely during measurement operation when in contact with an acid compound such as an acidic gas. Moreover, such mainly stationary reservoir of buffer keeps the concentration of dissolved buffer substance low, which in turn allows for the provision of a sensor with improved sensitivity. Thus, based on the particle and the composition according to the invention, an improved sensor element with high sensitivity and stability can be provided.

In a still further aspect, the present invention refers to a method for producing a sensor according to the invention, comprising:
a) providing a carrier,
b) applying the composition of the invention including at least one precursor of a hydrophobic polymer (xi) to the carrier, and
c) cross-linking the composition applied in step b) to obtain a hydrophobic polymer layer arranged on the carrier.

The carrier provided in step a) may be permeable to radiation, e.g. having a wavelength between 300 and 1300 nm. This allows for an excitation of the at least one detecting compound and optionally the at least one reference detecting compound applied to the carrier in subsequent step b) by radiation e.g. having a wavelength between 300 and 1300 nm. Moreover, emission and/or absorption characteristics of the at least one detecting compound and optionally the at least one reference compound e.g. in a range between 300 and 1300 nm may be detected.

In a preferred embodiment, the carrier is transparent. More particularly the carrier may be made of plastics, such as polyethylene terephthalate (PET), cyclic olefin copolymer (COC), polycarbonate, polystyrene, polyethylene (PE) or polypropylene (PP), or glass. The thickness of the carrier is e.g. 10-2000 µm, preferably 10-1000 µm, most preferred 50-125µm.

In step b) the composition may be applied to the carrier by a coating procedure such as spin coating, knife coating, spray coating, dip coating, or ink jet printing.

Step c) of cross-linking the composition applied in step b) may be performed by radical polymerization, condensation polymerization, addition polymerization, photo-initiated polymerization, or hydrosilylation. Preferably, step c) comprises irradiating the composition applied to the carrier, preferably using UV radiation.

The hydrophobic polymer layer obtained in step c) may have a thickness of 2-500 µm, preferably 10-125 µm. Particularly, the hydrophobic polymer layer obtained in step c) is permeable to radiation, e.g. having a wavelength between 300 and 1300 nm.

A hydrophobic polymer layer according to the invention particularly has a limited swelling capacity such as a swelling capacity of 1-20 wt.-% in water at 25 °C. The free volume of the hydrophobic polymer layer may be 0.01-0.50%, preferably 0.10-0.50% at 20°C. By this means, i.e. by providing a hydrophobic polymer layer having a swelling capacity of at most 20 wt.-% and/or a free volume of at least 0.01%, a hydrophobic polymer layer having a suitable water permeability is provided.

In another aspect, the present invention relates to a method of detecting an analyte, comprising the step of
α) contacting the analyte with the sensor or the composition of the invention,
β) irradiating the sensor or the composition of the invention with radiation of λ1 and optionally λ2,
γ) measuring the absorption, emission and/or fluorescence spectrum as signal,
δ) correlating the signal obtained in step γ) with the presence or absence of the analyte.

In step α) the compound to be detected is preferably present in aqueous medium which is in direct contact with the sensor or the composition of the invention. In this case, the swelling time in step α) preferably takes about 0-24 h, more preferably 6-24 h. In step β) the sensor or the composition of the invention is irradiated with radiation having a wavelength λ1 and optionally λ2 (step β). λ1 may be in the range of 300-1300 nm, preferably 400-1300nm. λ2 may be in the range of 300-1300nm, preferably 400-1300nm. Irradiation of the sensor with λ1 alone or in combination with λ2 depends on the detecting compound/reference detecting compound system used in the sensor or composition of the invention (see above).

The absorption, emission and/or fluorescence signal obtained may be measured by conventional spectrometer, such as a TECAN infinite M200 pro.

Correlation of the signal in step δ) may result in a qualitative or quantitative, more preferably quantitative, detection of the compound to be detected. Step δ) is preferably conducted via a calibrated data processing device for sensor elements as e.g. described in US 5,882,936. In this device, the analyte concentration in the sensor matrix can be calculated from the optical signal of the sensor recorded by optoelectronics.

The present invention shall be further illustrated in more detail but not limited by the following figures.

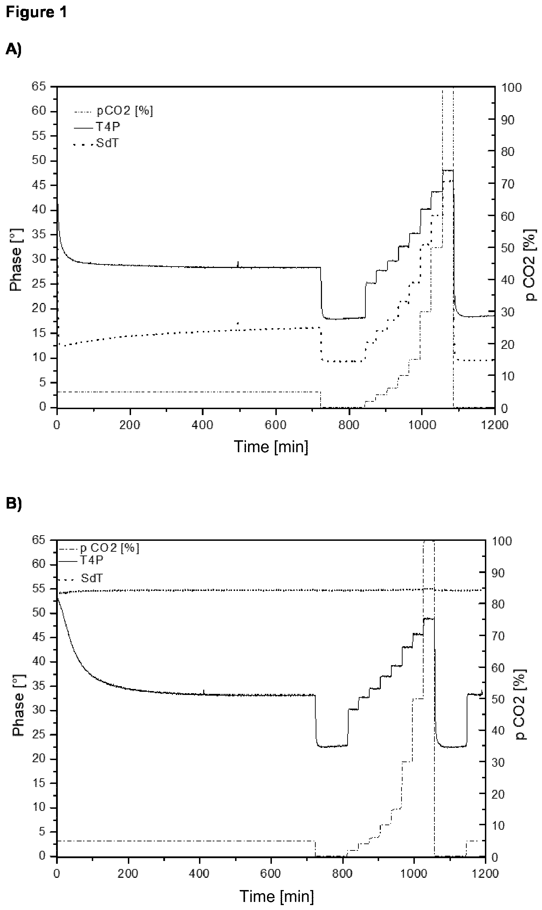

**Figure 1** displays the sensitivity of a sensor according to the invention (*T4P*) and an emulsoid based sensor according to the prior art (*SdT*) against varying carbon dioxide concentrations (pCO2) in sodium chloride solution with (Figure 1B) and without (Figure 1A) prior gamma sterilization.

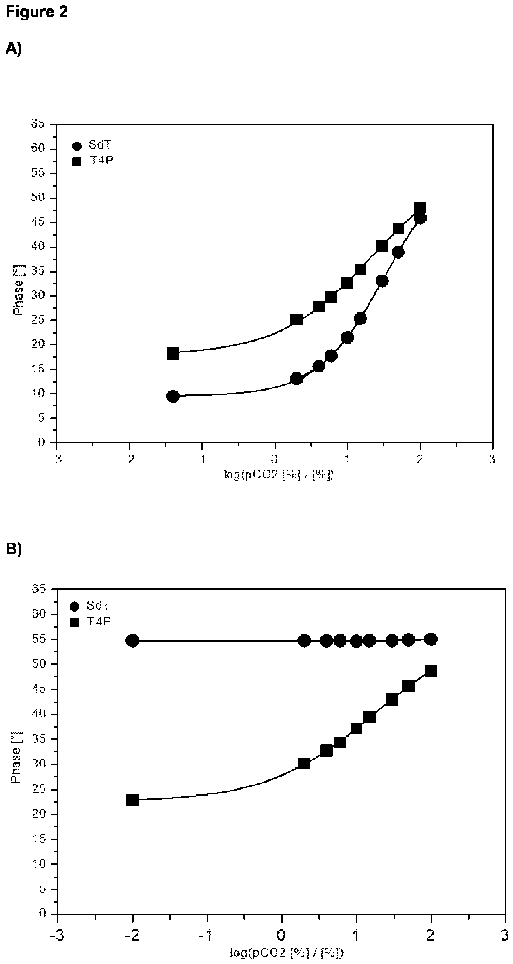

**Figure 2** displays calibration curves of a sensor according to the invention (*T4P*; squares) and an emulsoid based sensor according to the prior art (*SdT*; dots) with (Figure 2B) and without (Figure 2A) prior gamma sterilization as a function of the carbon dioxide concentration (pCO2) in 0.9 wt.-% sodium chloride solution.

The present invention shall be further illustrated in more detail but not limited by the following examples.

### Example 1: Preparation of particles according to the invention

In a 2.5 I flask, zinc chloride solution at a final concentration of 0.3 - 3.0 mol/l, aluminium chloride at a final concentration of 0.15 - 1.0 mol/l, urea solution at a final concentration of 2.0 - 5.0 mol/l, and 600 ml of water were stirred for 18 - 48 hours at 100 °C. The reaction mixture was then cooled and centrifuged. The particulate residue was washed with 300 ml of water for 3 hours under stirring to yield purified particles. The purified particles were stirred for 1 h in 1 l of 1 mol/l NaB (B = CO₃²⁻, PO₄³⁻, CO₃²⁻ : PO₄³⁻ = 1: 0,2; 1: 1; 1:2) solution, washed with water and dry to yield inorganically modified particles.

60-85 wt.-% of inorganically modified particles, 20-30 wt.-% of polyvinylpyrrolidone K15, 0.1-2 wt.-% of HPTS and 100 ml of water-free methanol were stirred for 1 h at room temperature followed by removal of the liquid medium using a rotary evaporator at 337 mbar and 40 °C. The resulting raw material was ground using a cryonic mill to yield particles according to the invention having an average particle size of 10 µm

### Example 2: Preparation of a sensor according to the invention

A mixture of 480 mg of particles obtained according to example 1, 360 mg of reference particles prepared according to EP 1 196 780 B1, and 4,7 g of vinyl terminated polydimethylsiloxane DMS V31, 400 mg of (methylhydrosiloxane)-dimethylsiloxane copolymer, trimethylsiloxane terminated, 25 - 35 cSt HMS 301 and 1,2 mg of (trimethyl)methylcyclopentadienylplatinum(IV) in 40 µl Hexane was stirred for 12 h at room temperature. Subsequently, the mixture was applied to a transparent PET carrier having a thickness of 125 µm by knife coating with a coating thickness of 20 µm. Then, the coating was cross-linked by UV radiation using a Iron Lamp (100 W/cm) nm) for 1 min.

### Comparative example 3: Preparation of an emulsoid based sensor

A sensor according to US 4,557,900 was prepared as follows: 25 mg of HPTS, 30 mg of Na₂CO₃, and 10 mg of Na₂HPO₄ were mixed with 1.0 g polyacrylamide (MW = 10 kDa) in 2.0 ml of water. The mixture obtained was dispersed in a mixture of 9.0 g of ELASTOSIL® RT 601 component A (vinyl-terminated polydimethylsiloxane), 1.0 g ELASTOSIL® RT 601 component B (cross-linker) and 300 mg of reference particles as described in Example 2. The dispersion was applied to a PET carrier having a thickness of 125 µm by knife coating with a coating thickness of 100 µm. Then, the coating was cross-linked for 48 h at 50 °C.

### Example 4: Sensor sensitivity and dynamics

A sensor according to example 2 and a comparative sensor according to example 3 were subjected to beta sterilization at 30 kGy in a 1L polycarbonate flask filled with air. Subsequently, their sensitivity against varying carbon dioxide concentrations (0.04, 2, 4, 6, 8, 10, 15, 30, 50, 100 %), corresponding to 0.04-100 %) in 0.9 % sodium chloride solution at 37 °C over a period of 20 h was analyzed (Figure 1B).

For the analysis the respective sensors were fixed at the distal end of polymer optical fibers and immersed in 0.9 wt.-% NaCl held at 37°C, the SMA-terminated fiber was connected to a PreSens EOM-pCO2-mini-optoelectronical-device. Via a gas-blender the respective gas-mixtures created from bottled gases for carbondioxide and nitrogen and were bubbled through the solution with the sensors. The sensor response - the phase value - was measured with an interval of 10 seconds with the optoelectronics and logged with a standard PC as described in WO1999/006821.

Furthermore, the sensors' sensitivity was analogously analyzed without prior sterilization (Figure 1A).

Although the sensor according to the invention *T4P* has a slightly lower sensitivity compared to comparative sensor SdT (Figure 1A) before the beta sterilization, afterwards it is still fully functional with only a minor decrease in sensitivity in contrast to the comparative sensor, which has completely lost its sensitivity and dynamics and thus was unable to detect the presence of carbon dioxide (Figure 1B).

Based on the data obtained by the above described sensitivity measurements of sterilized and non-sterilized sensors, calibration curves were calculated using a Boltzmann fit (Figure 2B and 2a, respectively). As can be seen from Figure 2A and table 1 below, the sensitivity, defined as the phase change per change in the CO₂-concentration for the sensor according to the invention T4P is slightly lower than for the comparative sensor SdT. For the comparative sensor SdT the phase difference before the sterilization between 0.04% CO₂- and 100% CO₂-saturation is 36.5°, for the sensor according to the invention T4P the phase difference between 0.04% pCO2 and 100% CO₂-saturation is 29.2°.

**Table 1. Boltzmann fit parameters (y = A2 + (A1-A2)/(1 + exp((x-x0)/dx))) for sensors T4P and SdT without prior sterilization.**

| Sensor | T4P | SdT |
|---|---|---|
| A1 | 17,90° | 9,61° |
| A2 | 58° | 58° |
| x0 | 1,32 | 1,50 |
| dx | 0,64 | 0,45 |

As can be seen from Figure 2B and table 2 below, after the beta sterilization the calibration curve for the comparative SdT sensor has flattened nearly completely with a remaining sensitivity of 0.25°, whereas the sensor according to the invention has a remaining sensitivity of 25.86° between 0.04% pCO₂ and 100% CO₂-saturation, enabling it - contrary to the comparative SdT-sensor- to measure the CO₂-concentration e.g. within an irradiation-presterilized biomedical device or bioreactor.

**Table 2. Boltzmann fit parameters (y = A2 + (A1-A2)/(1 + exp((x-x0)/dx))) for sensors T4P and SdT after gamma sterilization.**

| Sensor | T4P | SdT |
|---|---|---|
| A1 | 22,48° | 54,74° |
| A2 | 58° | 58° |
| x0 | 1,25 | 2,68 |
| dx | 0,72 | 0,30 |

The present invention covers the following items:
1. A particle, comprising
   (i) at least one inorganic compound having a buffer capacity of 0.1-50mmol/l at 20°C in a saturated, aqueous solution,
   (ii) at least one detecting compound, and
   (iii) at least one hygroscopic compound.
2. The particle according to item 1, wherein the inorganic compound (i) has a solubility of at most 1 g/l, preferably at most 0.5 g/l, more preferably 0.001-0.1 g/l, even more preferably 0,003 - 0.05 g/l in water at 20 °C and pH 7.
3. The particle according to any of the preceding items, wherein the inorganic compound (i) comprises phosphate ions, hydrogen phosphate ions, dihydrogen phosphate ions, carbonate ions, hydrogen carbonate ions, hydrogen sulfate ions, borate ions or a mixture thereof.
4. The particle according to any of the preceding items, wherein the inorganic compound (i) is selected from a carbonate salt, such as zinc carbonate or calcium carbonate, a phosphate salt or phosphate mineral, such as calcium phosphate or apatite, a hydrolysable metal oxide, such as magnesium oxide or zinc oxide, a layered mineral, such as a layered double hydroxide (LDH), or a mixture thereof.
5. The particle according to any of the preceding items, wherein the inorganic compound (i) is a modified layered mineral, preferably an inorganically modified layered mineral, such as an inorganically modified LDH.
6. The particle according to any of the items 4-5, wherein the layered mineral is modified by at least partially replacing the original interlayer anions of the layered mineral by inorganic anions other than the original interlayer anions, such as by phosphate ions, hydrogen phosphate ions, dihydrogen phosphate ions, carbonate ions, hydrogen carbonate ions, hydrogen sulfate ions, borate ions or a mixture thereof.
7. The particle according to any of the preceding items, wherein the detecting compound (ii) is a pH sensitive detecting compound, preferably a pH sensitive dye.
8. The particle according to any of the preceding items, wherein the detecting compound (ii) is pH sensitive in a range of at least ± 1 pH-unit to the pKa value of a compound to be detected.
9. The particle according to any of the preceding items, wherein the detecting compound (ii) comprises a fluorescence dye such as fluorescein, trisodium 8-hydroxypyrene-1,3,6-trisulfonate (HPTS), pyrenebutyric acid, 4-methylumbelliferone, acridine orange, disodium dihydroxypyrene disulfonate, SNAFL, SNARF, rhodamine B, aza-BODIPY, xylenol blue, m-cresol purple, thymolphthalein, carboxylfluorescein, 5'- and 6'-carboxyseminaphthofluorescein (c-SNAFL), 5'-(and 6')-carboxyseminaphthorhodafluorescein (c-SNARF), 7-hydroxycoumarin-4-acetic acid, tris(thenoyltrifluoroacetonato)europium (III), 1-hydroxypyrene-3,6,8-trisulfonic acid, neutral red, phenol red, rhodamine 6G, sulforhodamine 101, texas red hydrazide, bromocresol purple, eosin Y, 2',7'-dichlorofluorescein, oregon green, 3-substiuted 7-hydroxycoumarin or a salt or derivative thereof, preferably HPTS or a salt or derivative thereof.
10. The particle according to any of items 1-8, wherein the detecting compound (ii) comprises an absorption dye such as thymol blue, cresol red, phenol red, cresol purple, bromocresol green, phenolphthalein, o-cresolphthalein, bromothymol blue, bromophenol blue, 2-(2,4-dinitrophenylazo)-1-naphthol-3,6-disulfonic acid or a salt or derivative thereof, preferably cresol purple, thymol blue, or a salt or derivative thereof.
12. The particle according to any of the preceding items, wherein the detecting compound (ii) is insoluble in water.
13. The particle according to any of the preceding items, wherein the hygroscopic compound (iii) is an organic compound.
14. The particle according to any of the preceding items, wherein the hygroscopic compound (iii) is a homopolymer or copolymer, particularly a hydrophilic homopolymer or copolymer.
15. The particle according to any of the preceding items, wherein the hygroscopic compound (iii) comprises ionizable and/or ionic functional groups.
16. The particle according to any of the preceding items, wherein the hygroscopic compound (iii) is selected from polyvinylpyrrolidone (PVP), polyacrylamide, (hydroxyethyl)methacrylate (HEMA), cellulose derivatives such as carboxymethyl cellulose (CMC), methyl cellulose, ethyl cellulose, and hypromellose, agarose, dextran, poly(ethylene glycol), poly(propylene glycol), polyurethane, polyacrylic acid, or copolymers deriving from at least one of a (meth)acrylic acid, (hydroxyethyl)methacrylate (HEMA), vinylpyrrolidon monomeric unit, or a mixture thereof.
17. The particle according to any of the preceding items, wherein at least one detecting compound (ii) is covalently coupled to at least one hygroscopic compound (iii).
18. The particle according to any of the preceding items, wherein at least one detecting compound (ii) is coupled to at least one hygroscopic compound (iii) via coulomb interactions.
19. The particle according to any of the preceding items, wherein the particle is a core-shell-particle.
20. The particle according to any of the preceding items, wherein the shell comprises the at least one detecting compound (ii) and the at least one hygroscopic compound (iii).
21. The particle according to any of the preceding items, wherein the core comprises the at least one inorganic compound (i).
22. The particle according to any of the preceding items, wherein the particle has an average particle size of up to 500 µm, preferably up to 100 µm, more preferably 0.01-10 µm, even more preferably 0.01-1µm.
23. The particle according to any of the preceding items, wherein the amount of the at least one inorganic compound (i) is 50-90 wt.-%, preferably 60-84 wt.-% with reference to the total weight of the particle.
24. The particle according to any of the preceding items, wherein the amount of the at least one detecting compound (ii) is 0.1-5 wt.-%, preferably 0.1-2 wt.-% with reference to the total weight of the particle.
25. The particle according to any of the preceding items, wherein the amount of the at least one hygroscopic compound (iii) is 10-30 wt.-%, preferably 20-30 wt.-% with reference to the total weight of the particle.
26. A method for producing the particle according to any of items 1-25, comprising:
   a) providing a mixture comprising at least one detecting compound (ii), at least one hygroscopic compound (iii) and at least one liquid medium,
   b) applying the mixture of step a) to at least one inorganic compound (i), and
   c) optionally at least partially removing the at least one liquid medium from the mixture obtained in step b),
   wherein the at least one inorganic compound (i) has a buffer capacity of 0.1-50 mmol/l at 20 °C in a saturated, aqueous solution.
27. The method according to item 26, wherein the mixture obtained in step a) is essentially free from water.
28. The method according to any of items 26-27, wherein the at least one liquid medium is essentially free from water.
29. The method according to any of items 26-28, wherein the liquid medium is an organic solvent, preferably an alcohol, such as methanol, ethanol or propanol, tetrahydrofuran, 1,4-dioxane, dichloromethane, acetonitrile, acetone, dimethylformamide, or N-methylpyrrolidone.
30. The method according to any of items 26-29, wherein the detecting compound (ii) is a pH sensitive detecting compound, preferably a pH sensitive dye.
31. The method according to any of items 26-30, wherein the detecting compound (ii) is pH sensitive in a range of at least ± 1 pH-unit to the pKa value of a compound to be detected.
32. The method according to any of items 26-31, wherein the detecting compound (ii) comprises a fluorescence dye such as fluorescein, trisodium 8-hydroxypyrene-1,3,6-trisulfonate (HPTS), pyrenebutyric acid, 4-methylumbelliferone, acridine orange, disodium dihydroxypyrene disulfonate, SNAFL, SNARF, rhodamine B, aza-BODIPY, xylenol blue, m-cresol purple, thymolphthalein, carboxylfluorescein, 5'- and 6'-carboxyseminaphthofluorescein (c-SNAFL), 5'-(and 6')-carboxyseminaphthorhodafluorescein (c-SNARF), 7-hydroxycoumarin-4-acetic acid, tris(thenoyltrifluoroacetonato)europium (III), 1-hydroxypyrene-3,6,8-trisulfonic acid, neutral red, phenol red, rhodamine 6G, sulforhodamine 101, texas red hydrazide, bromocresol purple, eosin Y, 2',7'-dichlorofluorescein, oregon green, 3-substiuted 7-hydroxycoumarin or a salt or derivative thereof.
33. The method according to any of items 26-31, wherein the detecting compound (ii) comprises an absorption dye such as thymol blue, cresol red, phenol red, cresol purple, bromocresol green, phenolphthalein, o-cresolphthalein, bromothymol blue, bromophenol blue, 2-(2,4-dinitrophenylazo)-1-naphthol-3,6-disulfonic acid or a salt or derivative thereof.
34. The method according to any of items 26-33, wherein the detecting compound (ii) is essentially insoluble in water.
35. The method according to any of items 26-34, wherein the hygroscopic compound (iii) is an organic compound.
36. The method according to any of items 26-35, wherein the hygroscopic compound (iii) is a homopolymer or copolymer, particularly a hydrophilic homopolymer or copolymer.
37. The method according to any of items 26-36, wherein the hygroscopic compound (iii) comprises ionic and/or ionizable functional groups.
38. The method according to any of items 26-37, wherein the hygroscopic compound (iii) is selected from polyvinylpyrrolidone (PVP), polyacrylamide, (hydroxyethyl)methacrylate (HEMA), cellulose derivatives such as carboxymethyl cellulose (CMC), methyl cellulose, ethyl cellulose and hypromellose, agarose, dextran, poly(ethylene glycol), poly(propylene glycol), polyurethane, polyacrylic acid, or copolymers deriving from at least one of a (meth)acrylic acid, (hydroxyethyl)methacrylate (HEMA), or vinylpyrrolidone monomeric unit, or a mixture thereof.
39. The method according to any of items 26-38, wherein the inorganic compound (i) has a solubility of at most 1 g/l, preferably at most 0.5 g/l, more preferably 0.001-0.1 g/l, even more preferably 0,003 - 0.05 g/l in water at 20 °C and pH 7.
40. The method according to any of items 26-39, wherein the inorganic compound (i) comprises phosphate ions, hydrogen phosphate ions, dihydrogen phosphate ions, carbonate ions, hydrogen carbonate ions, borate ions, hydrogen sulfate ions or a mixture thereof.
41. The method according to any of items 26-40, wherein the inorganic compound (i) is selected from a carbonate salt, such as zinc carbonate or calcium carbonate, a phosphate salt or mineral, such as calcium phosphate or apatite, a hydrolysable metal oxide, such as magnesium oxide or zinc oxide, a layered mineral, such as a layered double hydroxide (LDH), or a mixture thereof.
42. The method according to any of items 26-41, wherein the inorganic compound (i) is a modified layered mineral, preferably an inorganically modified layered mineral, such as an inorganically modified LDH.
43. The method according to any of items 26-42, wherein the layered mineral is modified by at least partially replacing the original interlayer anions of the layered mineral by inorganic anions other than the original interlayer anions, such as by phosphate ions, hydrogen phosphate ions, dihydrogen phosphate ions, carbonate ions, hydrogen carbonate ions, borate ions, hydrogen sulfate ions, or a mixture thereof.
44. The method according to any of items 26-43, wherein the ratio of the at least one detecting compound (ii) to the at least one hygroscopic compound (iii) in step a) is 1:10 to 1:1, preferably 1:5 to 3:10.
45. The method according to any of items 26-44, wherein the at least one inorganic compound (i) provided in step b) is present in the form of solid particles.
46. The method according to any of items 26-45, wherein step b) is performed by mixing the at least one inorganic compound (i) and the mixture provided in step a) in a reaction vessel.
47. The method according to any of items 26-46, wherein step b) is performed by atomizing the mixture provided in step a) onto or around the at least one inorganic compound (i).
48. The method according to any of items 26-47, wherein the ratio of the at least one inorganic compound (i) to the at least one hygroscopic compound (iii) in step b) is 3:10 to 1:1, preferably 1:3 to 2:5
49. The method according to any of items 26-48, wherein the ratio of the at least one inorganic compound (i) to the at least one detecting compound (ii) in step b) is 1:1 to 10:1, preferably 3:2 to 6:1
50. The method according to any of items 26-49, wherein step b) is performed at temperatures of 20-80°C.
51. The method according to any of items 26-50, wherein in step c) at least 90%, preferably at least 95%, more preferably at least 99% of the at least one liquid medium is removed.
52. The method according to any of items 26-51, wherein step c) is performed at elevated temperature, such 20-80°C, preferably at 40-60 °C, and/or at reduced pressure, preferably below 1 bar, more preferably below 500 mbar.
53. The method according to any of items 26-52, further comprising the step:
   d) grinding the mixture obtained in step b) or optionally in step c).
54. A composition comprising:
   - particles according to any of items 1-25, and
   - (x) at least one hydrophobic polymer or (xi) at least one precursor thereof.
55. The composition according to item 54, comprising at least one reference detecting compound.
56. The composition according to any of items 54-55, wherein the hydrophobic polymer (x) is cross-linked.
57. The composition according to any of items 54-56, wherein the hydrophobic polymer (x) is permeable to radiation e.g. having a wavelength between 300 and 1300 nm.
58. The composition according to any of items 54-57, wherein the hydrophobic polymer (x) has a swelling capacity of 0.01-10 wt.-% in water at 25 °C.
59. The composition according to any of items 54-58, wherein the hydrophobic polymer (x) has a free volume of 0.01-0.50%, preferably 0.10-0.50% at 20°C.
60. The composition according to any of items 54-59, wherein the hydrophobic polymer (x) is selected from a silicone polymer such as polydimethylsiloxane (PDMS) or a fluorinated silicone polymer, or a silicone copolymer such as a PDMS copolymer, a silicone-polycarbonate copolymer or a fluorinated silicone copolymer.
61. The composition according to any of items 54-60, wherein the at least one precursor (xi) comprises at least one monomer capable of forming a hydrophobic polymer (x) and optionally an initiator, a catalyst, a cross-linker or a mixture thereof.
62. The composition according to any of items 54-61, wherein the monomer is selected from vinyl terminated poly(dimethylsiloxane), vinylphenylmethyl terminated (phenylmethylsiloxane)-vinylphenylsiloxane copolymer, vinyl terminated diphenylsiloxane-dimethylsiloxane copolymers, vinyl terminated trifluoropropylmethylsiloxane-dimethylsiloxane copolymer, vinyl terminated nonafluorohexylmethylsiloxane-dimethylsiloxane copolymer, vinyl terminated diethylsiloxane-dimethylsiloxane copolymers, vinyl terminated ethylene-siloxane copolymer fluids, and mixtures thereof.
63. The composition according to any of items 54-62, wherein the initiator is selected from benzoyl peroxide, cumene hydroperoxide, and mixtures thereof.
64. The composition according to any of items 54-63, wherein the catalyst is selected from photo-active precious metal catalysts such as (trimethyl)methylcyclopentadienylplatinum(IV) or tris(dibutylsulfide)rhodium trichloride, or non-photoinitiated catalysts like Karstedt's catalyst or platinum-divinyltetramethyldisiloxane complex.
65. The composition according to any of items 54-64, wherein the cross-linker is selected from (methylhydrosiloxane)-dimethylsiloxane copolymers, trimethylsiloxy terminated polydimethylsiloxanes, hydride terminated polydimethylsiloxanes, and mixtures thereof.
66. The composition according to any of items 54-65, wherein the amount of particles is 5-50 wt.-%, preferably 10-20 wt.-% with reference to the total weight of the composition.
67. The composition according to any of items 54-66, wherein the amount of the at least one reference detecting compound is 2-20 wt.-%, preferably 3-15 wt.-% with reference to the total weight of the composition.
68. The composition according to any of items 54-67, wherein the weight ratio of particles according to any of item 1-25 to reference particles is 10:1 - 1:1, preferably 2:1 - 1:1.
69. The composition according to any of items 54-68, wherein the amount of polymer (x) or precursor (xi) is 48-95 wt.-%, preferably 48-90 wt.-% with reference to the total weight of the composition.
70. Use of a composition according to any of items 54-69 as a sensor.
71. The use according to item 70, wherein the sensor is a gas sensor, particularly a CO₂ gas sensor.
72. A sensor, comprising the composition according to any of items 54(x)-69.
73. A method for producing a sensor according to item 72, comprising:
   a) providing a carrier,
   b) applying the composition according to any of items 54(xi)-69 to the carrier, and
   c) cross-linking the composition applied in step b) to obtain a polymer layer arranged on the carrier.
74. The method according to item 73, wherein the carrier is permeable to radiation having a wavelength between 300 and 1300 nm.
75. The method according to any of items 73-74, wherein the carrier is made of plastics, such as polyethylene terephthalate (PET), cyclic olefin copolymer (COC), polycarbonate, polystyrene, polyethylene (PE), polypropylene (PP), or glass.
76. The method according to any of items 73-75, wherein the carrier is transparent.
77. The method according to any of items 73-76, wherein the carrier has a thickness of 10-2000 µm, preferably 10-1000 µm.
78. The method according to any of items 73-77, wherein step b) is performed by a coating procedure such as spin coating, knife coating, spray coating, dip coating, or ink jet printing.
79. The method according to any of items 73-78, wherein step c) is performed by radical polymerization, condensation polymerization, addition polymerization, photo-initiated polymerization, or hydrosilylation.
80. The method according to any of items 73-79, wherein step c) comprises irradiating the composition applied to the carrier, preferably using UV radiation.
81. The method according to any of items 73-80, wherein the polymer layer obtained in step c) has a thickness of 2-500 µm, preferably 10-50 µm.
82. The method according to any of items 73-81, wherein the polymer layer obtained in step c) is permeable to radiation, preferably having a wavelength between 300 and 1300 nm.
83. The method according to any of items 73-82, wherein the polymer layer obtained in step c) has a swelling capacity of 1-20 wt.-% in water at 25 °C.
84. The method according to any of items 73-83, wherein the polymer layer obtained in step c) has a free volume of 0.01-0.50% at 20 °C.
85. Method of detecting an analyte compound, comprising the step of
   α) contacting the analyte with the sensor according to item 72 or the composition according to any of items 54-69,
   β) irradiating the sensor or the composition with radiation λ1 and optionally λ2,
   γ) measuring the absorption, emission and/or fluorescence spectrum as signal,
   δ) correlating the signal obtained in step γ) with the presence or absence of the analyte.

## Claims

1. A particle, comprising
(i) at least one inorganic compound having a buffer capacity of 0.1-50mmol/l at 20°C in a saturated, aqueous solution,
(ii) at least one detecting compound, and
(iii) at least one hygroscopic compound.

2. The particle according to claim 1, wherein the at least one inorganic compound (i) has a solubility of at most 1 g/l in water at 20 °C and pH 7, and is preferably selected from a carbonate salt, such as zinc carbonate or calcium carbonate, a phosphate salt or mineral, such as calcium phosphate or apatite, a hydrolysable metal oxide, such as magnesium oxide or zinc oxide, a layered mineral, such as a layered double hydroxide (LDH), or a mixture thereof.

3. The particle according to any of the preceding claims, wherein the at least one detecting compound (ii) is a pH sensitive detecting compound, preferably a pH sensitive dye.

4. The particle according to any of the preceding claims, wherein the at least one hygroscopic compound (iii) is a homopolymer or copolymer, particularly a hydrophilic homopolymer or copolymer, particularly comprising ionizable and/or ionic functional groups.

5. A method for producing the particle according to any of items 1-25, comprising:
a) providing a mixture comprising at least one detecting compound (ii), at least one hygroscopic compound (iii) and at least one liquid medium,
b) applying the mixture of step a) to at least one inorganic compound (i), and
c) optionally at least partially removing the at least one liquid medium from the mixture obtained in step b),
wherein the at least one inorganic compound (i) has a buffer capacity of 0.1-50 mmol/l at 20 °C in a saturated, aqueous solution.

6. The method according to claim 5, wherein the at least one liquid medium is an organic solvent, preferably an alcohol, such as methanol, ethanol or propanol, tetrahydrofuran, 1,4-dioxane, dichloromethane, acetone, dimethylformamide, or N-methylpyrrolidone.

7. A composition comprising:
- particles according to any of claims 1-4, and
- (x) at least one hydrophobic polymer or (xi) at least one precursor thereof.

8. The composition according to claim 7, comprising a reference detecting compound.

9. The composition according to any of claims 7-8, wherein the hydrophobic polymer (x) is selected from a silicone polymer such as polydimethylsiloxane (PDMS) or a fluorinated silicone polymer, or a silicone copolymer such as a PDMS copolymer, a silicone-polycarbonate copolymer or a fluorinated silicone copolymer.

10. The composition according to any of claims 7-9, wherein the at least one precursor (Xi) comprises at least one monomer capable of forming a hydrophobic polymer (x) and optionally an initiator, a catalyst, a cross-linker or a mixture thereof, the monomer being e.g. selected from vinyl terminated poly(dimethylsiloxane), vinylphenylmethyl terminated (phenylmethylsiloxane)-vinylphenylsiloxane copolymer, vinyl terminated diphenylsiloxane-dimethylsiloxane copolymers, vinyl terminated trifluoropropylmethylsiloxane-dimethylsiloxane copolymer, vinyl terminated nonafluorohexylmethylsiloxane-dimethylsiloxane copolymer, vinyl terminated diethylsiloxane-dimethylsiloxane copolymers, vinyl terminated ethylene-siloxane copolymer fluids, and mixtures thereof.

11. Use of a composition according to any of claims 7-10 as a sensor, particularly a CO₂ gas sensor.

12. A sensor, comprising the composition according to any of claims 7(x)-11.

13. A method for producing a sensor according to claim 12, comprising:
a) providing a carrier,
b) applying the composition according to any of claims 7(xi)-10 to the carrier, and
c) cross-linking the composition applied in step b) to obtain a polymer layer arranged on the carrier.

14. The method according to claim 13, wherein step b) is performed by a coating procedure such as spin coating, knife coating, spray coating, dip coating, or ink jet printing.

15. Method of detecting an analyte, comprising the step of
α) contacting the analyte with the sensor according to claim 12 or the composition according to any of claims 7-10,
β) irradiating the sensor or the composition with radiation λ1 and optionally λ2,
γ) measuring the absorption, emission and/or fluorescence spectrum as signal,
δ) correlating the signal obtained in step γ) with the presence or absence of the analyte.
